# EUROPEAN PATENT SPECIFICATION

(11) **EP 1 353 942 B1**
(45) Date of publication and mention of the grant of the patent: **15.12.2010**
(21) Application number: 01973355.9
(22) Date of filing: 21.09.2001
(51) Int. Cl.: C07K 14/16

(54) **PEPTIDE INHIBITORS OF HIV ENTRY**
PEPTIDINHIBITOREN GEGEN DEN EINTRITT VON HIV IN ZELLEN
INHIBITEURS PEPTIDIQUES DE LA PENETRATION DU VIH

(30) Priority: 22.09.2000 US 668072
(43) Date of publication of application: 22.10.2003
(73) Proprietor: WHITEHEAD INSTITUTE FOR BIOMEDICAL RESEARCH, Cambridge, MA 02142 (US)
(72) Inventor: ECKERT, Debra, M., Scotch Plains, NJ 07076 (US); SUNTOKE, Tara, R., Pasadena, CA 91107 (US); KIM, Peter, S., Bryn Mawr, PA 19010 (US)
(74) Representative: Lock, Graham James
(86) International application number: PCT/US2001/029637
(87) International publication number: WO 2002/024735

(56) References cited:
- WO-A-00/06599
- WO-A-00/40616
- CHAN D C ET AL: "Evidence that a prominent cavity in the coiled coil of HIV type 1 gp41 is an attractive drug target" PROCEEDINGS OF THE NATIONAL ACADEMY OF SCIENCES OF USA, NATIONAL ACADEMY OF SCIENCE. WASHINGTON, US, vol. 95, December 1998 (1998-12), pages 15613-15617, XP002126805 ISSN: 0027-8424
- ECKERT D M ET AL: "INHIBITING HIV-1 ENTRY: DISCOVERY OF D-PEPTIDE INHIBITORS THAT TARGET THE GP41 COILED-COIL POCKET" CELL, CELL PRESS, CAMBRIDGE, NA, US, vol. 99, no. 1, 1 November 1999 (1999-11-01), pages 103-115, XP000982264 ISSN: 0092-8674
- YANG XINZHEN ET AL: "Characterization of stable, soluble trimers containing complete ectodomains of human immunodeficiency virus type 1 envelope glycoproteins." JOURNAL OF VIROLOGY, vol. 74, no. 12, June 2000 (2000-06), pages 5716-5725, XP002222730 ISSN: 0022-538X
- JIANG SHIBO ET AL: "Development of HIV entry inhibitors targeted to the coiled-coil regions of gp41." BIOCHEMICAL AND BIOPHYSICAL RESEARCH COMMUNICATIONS, vol. 269, no. 3, 24 March 2000 (2000-03-24), pages 641-646, XP002222731 ISSN: 0006-291X
- ECKERT DEBRA M ET AL: "Crystal structure of GCN4-pIQI, a trimeric coiled coil with buried polar residues." JOURNAL OF MOLECULAR BIOLOGY, vol. 284, no. 4, 11 December 1998 (1998-12-11), pages 859-865, XP002222732 ISSN: 0022-2836 cited in the application
- SINGH MONA ET AL: "LearnCoil-VMF: Computational evidence for coiled-coil-like motifs in many viral membrane-fusion proteins." JOURNAL OF MOLECULAR BIOLOGY, vol. 290, no. 5, 30 July 1999 (1999-07-30), pages 1031-1041, XP002222733 ISSN: 0022-2836 cited in the application
- SUZUKI KAZUO ET AL: "An isoleucine zipper peptide forms a native-like triple stranded coiled coil in solution" PROTEIN ENGINEERING, OXFORD UNIVERSITY PRESS, SURREY, GB, vol. 11, no. 11, 1 November 1998 (1998-11-01), pages 1051-1055, XP009103953 ISSN: 0269-2139
- TANAKA, TOSHIKI; SUZUKI, KAZUO: "Design of a triple stranded coiled coil exhibiting a pH dependent conformational change" PEPTIDE SCIENCE, vol. 35, 1999, pages 389-392, ISSN: 1344-7661
- SUZUKI KAZUO ET AL: "An isoleucine zipper peptide forms a native-like triple stranded coiled coil in solution" PROTEIN ENGINEERING, OXFORD UNIVERSITY PRESS, SURREY, GB, vol. 11, no. 11, 1 November 1998 (1998-11-01), pages 1051-1055, XP009103953 ISSN: 0269-2139
- TANAKA, TOSHIKI; SUZUKI, KAZUO: "Design of a triple stranded coiled coil exhibiting a pH dependent conformational change" PEPTIDE SCIENCE, vol. 35, 1999, pages 389-392, ISSN: 1344-7661

## Description

### BACKGROUND OF THE INVENTION

HIV is the virus that is responsible for the worldwide AIDS epidemic. The initial stages of HIV infection involve the fusion of viral membrane with the target cell membrane, a process that injects the viral contents into the cellular cytoplasm. On the viral side, the molecular complex responsible for the fusion activity contains the surface protein gp 120 and the transmembrane protein gyp41. It is the current hypothesis that gp120 interacts with the proteins CD4 and coreceptor on the target cell, resulting in a conformational change that causes gp41 to insert its amino terminus (fusion peptide region) into the target cell membrane. This structural rearrangement promotes the fusion of virus and cellular membranes through a poorly understood mechanism.

### SUMMARY OF THE INVENTION

Described herein are chimeric peptides comprising a soluble trimeric coiled-coil and all or a portion of the N-peptide region of HIV gp41. These molecules are stable, trimeric coiled-coils that inhibit HIV entry into cells, such as human cells. Such peptides can be further assessed to demonstrate their ability to serve as potent anti-HIV therapeutic molecules and thus, as therapeutic molecules or drugs.

WO 00/06599 and Eckert et al. (1999) Inhibiting HIV-1 entry: discovery of D-peptide inhibitors that target the gp41 coiled-coil pocket. Cell 99(1):103-115 describe soluble fusion peptides comprising various portions of the N-terminal region of gp41.

### BRIEF DESCRIPTION OF THE DRAWINGS

The file of this patent contains at least one drawing executed in color. Copies of this patent with color drawing(s) will be provided by the Patent and Trademark Office upon request and payment of the necessary fee.

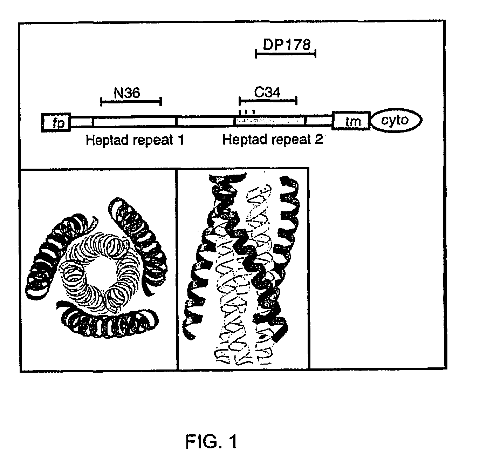

Figure 1 is the structural arrangement of HIV gp41. Helical regions (heptad repeats) are shown in grey and the relative position of N-(N36) and C-(C34, DP178) peptides are indicated. In the ribbon diagram of the helical region, the N-peptides are in light grey and the C-peptides are in dark grey.

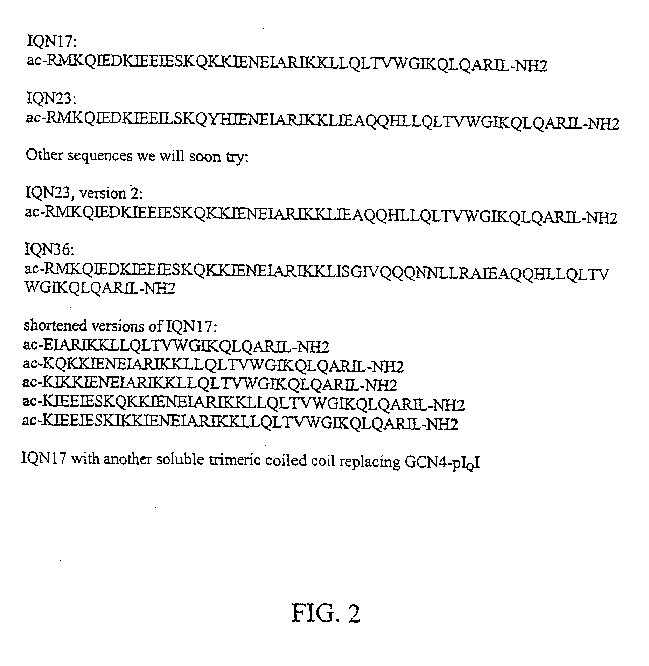

Figure 2 is the amino acid sequences of IQ peptides (SEQ ID Nos: 1-9).

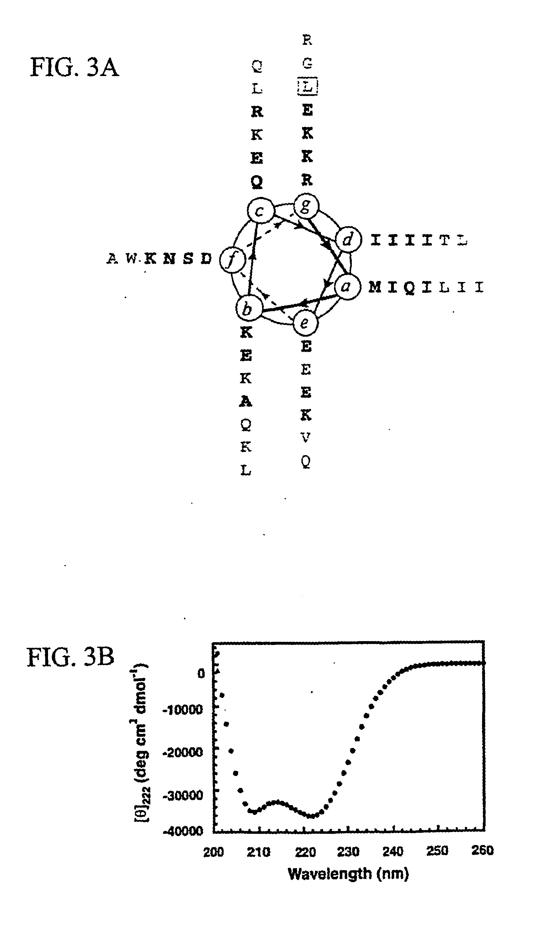

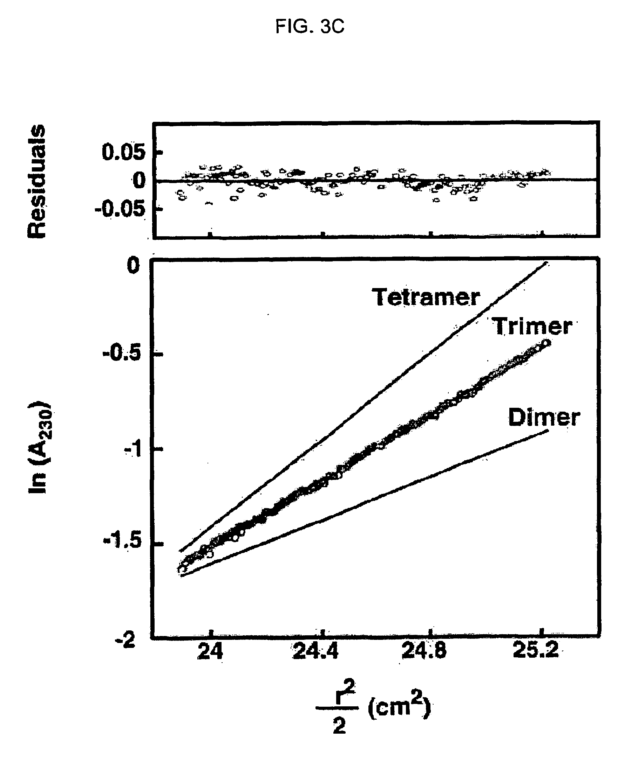

Figures 3A - 3B show, respectively, a helical wheel representation of IQN17 (Figure 3A); the CD spectrum of IQN17 (Figure 3B); and analytical ultracentrifugation data for IQN17 (Figure 3C). "XLA" is referred to herein as analytical ultracentrifugation.

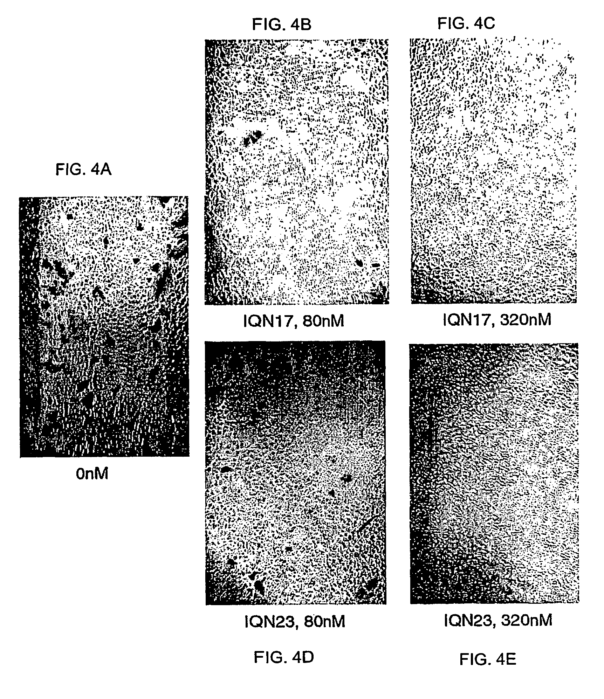

Figures 4A - 4E are photographs of results of syncytia assays carried out in the absence of IQN peptide (Figure 4A), in the presence of IQN17 at 80 nM (Figure 4B) or at 320nM (Figure 4C) or in the presence of IQN23 at 80 nM (Figure 4D) or at 320 nM (Figure 4E).

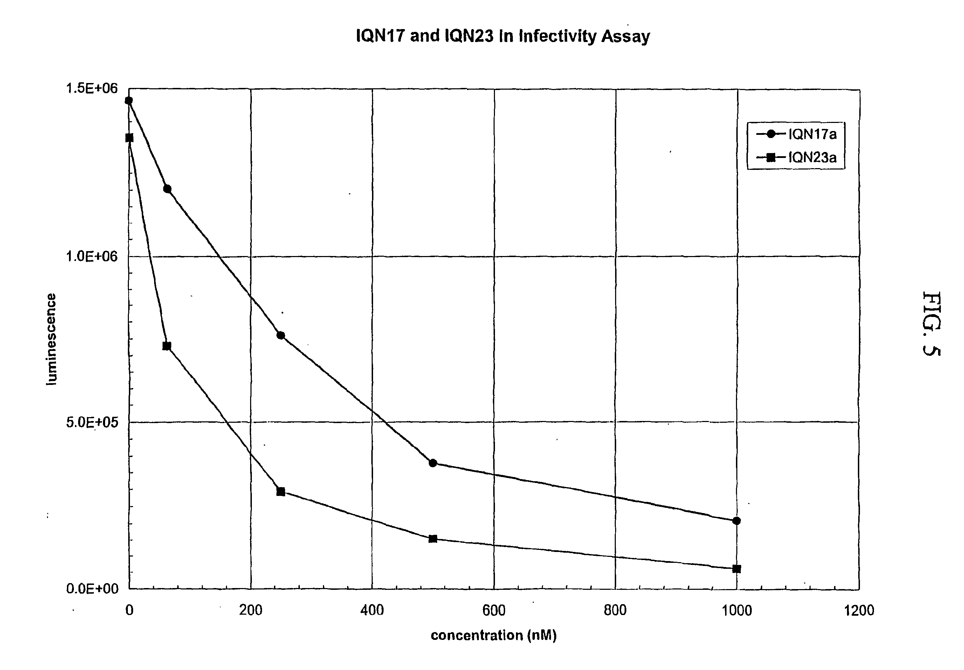

Figure 5 is a graphic representation of the inhibitory activity of IQN17 and IQN23 in a viral infectivity assay.

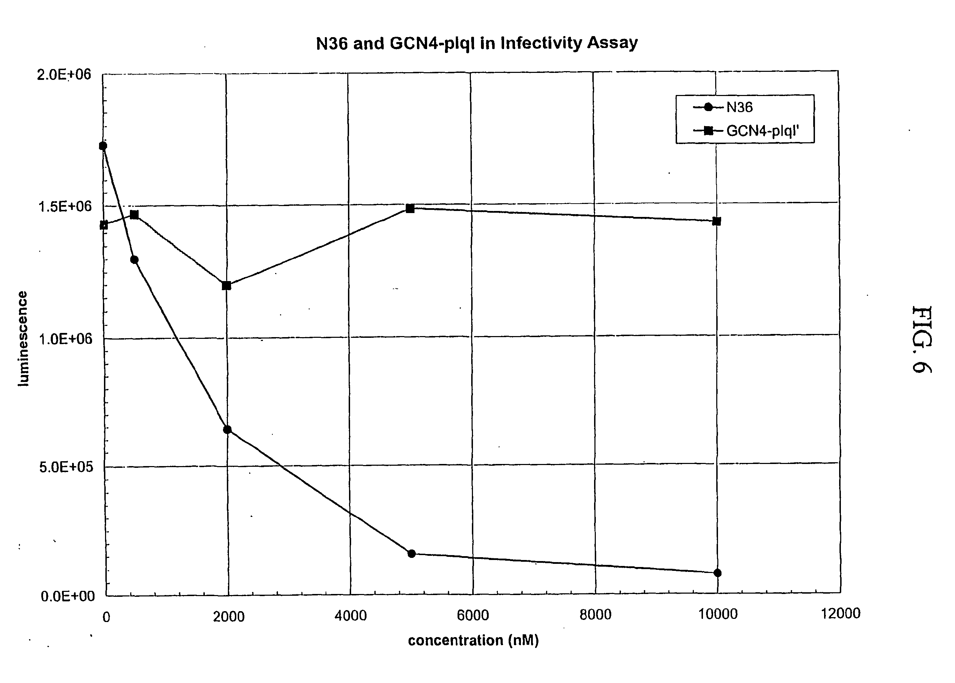

Figure 6 is a graphic representation of the inhibitory activity of N36 and GCN4-pI_{Q} I in a viral infectivity assay. The results presented clearly show a lack of inhibitory activity by both N36 and GCN-pI_{Q}I.

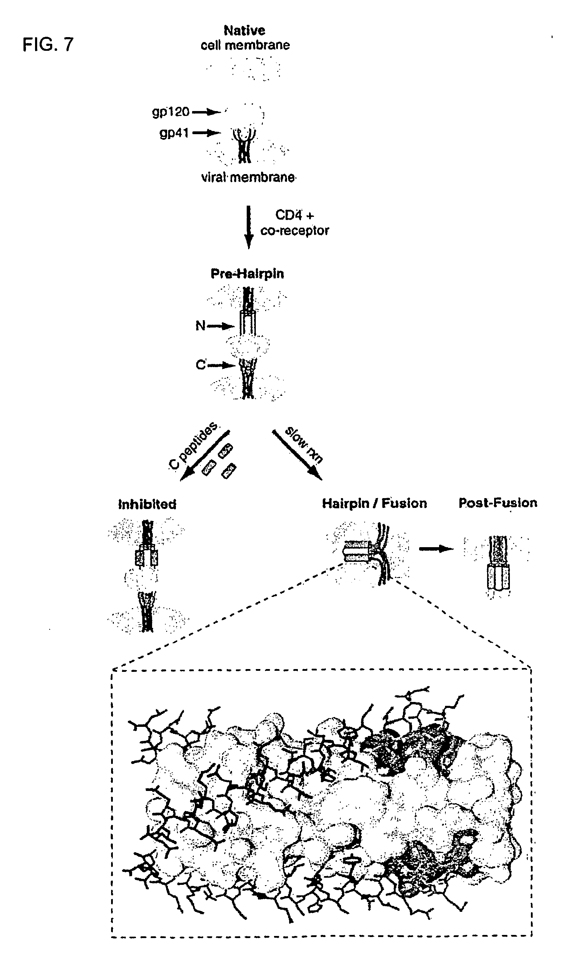

Figure 7 is a working model for HIV membrane fusion (Chan & Kim 1988). In the native state of HIV-1 env ("Native"), the fusion-peptide and N-peptide regions of gp41 are not exposed. Following interaction with cellular receptors (CD4 and coreceptor), a conformational change results in formation of the transient pre-hairpin intermediate ("Pre-hairpin"), in which the fusion-peptide regions (red lines) are inserted into the cell membrane and the coiled coil of the N-peptide region of gp41 (indicated as "N") is exposed. However, the C-peptide region of gp41 (indicated as "C") is constrained and unavailable for interaction with the coiled coil. Thus, exogenous C-peptides can bind to the pre-hairpin intermediate and inhibit fusion in a dominant-negative manner ("Inhibited"). In the absence of inhibitors, the pre-hairpin intermediate resolves to the hairpin structure and membrane fusion results ("Hairpin/Fusion"), although it is not known if hairpin formation precedes membrane fusion per se. The inset depicts the 2.0Å X-ray crystal structure of N36/C34, a peptide version of the HIV-1 gp41 core (Chan et al. 1997). Three central N-peptides form a coiled coil, shown here as a surface representation, and three helical C-peptides pack along conserved grooves on the surface of the coiled coil trimer. There are three symmetry-related hydrophobic pockets on the surface of the N-peptide coiled coil (shaded).

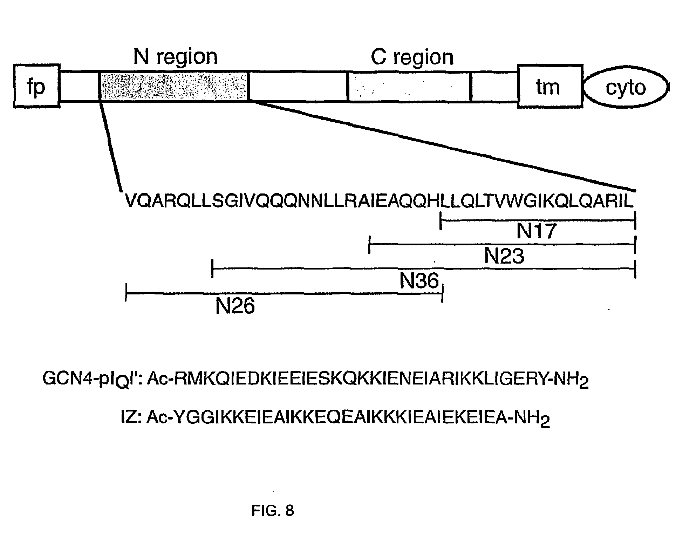

Figure 8 is a graphic illustration showing that the chimeric peptides are composed of two parts: 1) one of the designed trimeric coiled coils (GCN4-pI_{Q}I or IZ) (SEQ ID Nos: 10 and 11) and 2) one of the four regions of gp41 (SEQ ID NO: 12).

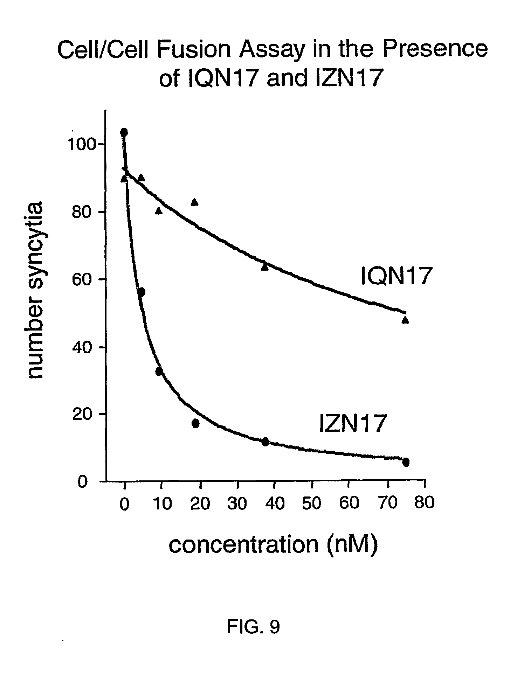

Figure 9 is a graph of concentration (nM) versus number syncytium showing the results of the cell/cell fusion assay in the presence of IQN17 and IZN17.

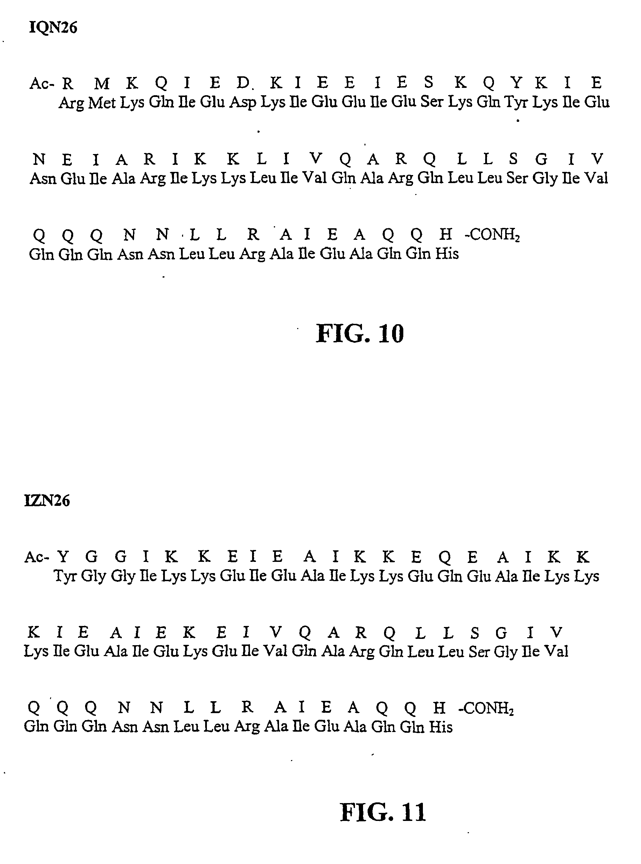

Figure 10 is the amino acid sequence of IQN26 (SEQ ID NO: 13).
Figure 11 is the amino acid sequence of IZN26 (SEQ ID NO: 14).

### DETAILED DESCRIPTION OF THE INVENTION

The present invention relates to soluble peptides as defined in the claims. Under the conditions described herein, the peptides fold into a stable trimeric coiled-coil (helical) structure and inhibit HIV infection of mammalian cells, such as human cells.

The "IZ" molecule derived from Tanaka et al., but with mutations: Ac-YGGIKKEIEAIKKEQEAIKKKIEAIEKEIEA-NH2 (SEQ ID NO: 11).

The IZ peptides of the present invention can be produced as a continuous peptide or as components that are joined or linked after they are formed. As used herein, the terms "joined" or "joined in such a manner" or "incorporated" include incorporating amino acid residues by either approach.

Changes can also be made in the amino acid composition of the fusion protein component which is the C-terminal portion of the HIV gp41 N peptide to produce a variety of fusion proteins to be used to prevent HIV infection of cells. The C-terminal portion can be changed by the addition, substitution, modification and/or deletion of one or more amino acid residues. The amino acid composition of either or both components of the fusion protein can be altered, and there is no limit to the number or types of amino acid residue changes possible, provided that the trimeric state of the coiled-coil is maintained. It is not necessary that the pocket or cavity of gp41 be included, although in many embodiments the pocket is present.

In all embodiments, controlled or time release (gradual release, release at a particular time after administration or insertion) of the drug can be effected by, for example, incorporating the drug into a composition which releases the drug gradually or after a defined period of time. Alternatively, the drug can be incorporated into a composition which releases the drug immediately or soon after its administration or application (e.g., into the blood, vagina, mouth or rectum). Combined release (e.g., release of some of the drug immediately or soon after insertion, and over time or at a particular time after insertion) can also be effective (e.g., by producing a composition which is comprised of two or more materials: one from which release or delivery occurs immediately or soon after insertion and/or one from which release or delivery is gradual and/or one from which release occurs after a specified period). For example, a drug or drugs which bind the HIV cavity can be incorporated into a sustained release composition such as that taught in U.S. Patent 4,707,362. The cream, foam, gel or suppository can be one also used for birth control purposes (e.g., containing a spermicide or other contraceptive agent), although that is not necessary (e. a., it can be used solely to deliver the anti-HIV drug, alone or in combination with another non-contraceptive agent, such as an antibacterial or antifungal drug or a lubricating agent). An anti-HIV drug of the present invention can also be administered to an individual through the use of a contraceptive device (e.g., condom, cervical cap, diaphragm) which is coated with or has incorporated therein in a manner which permits release under conditions of use a drug or drugs which bind the HIV gp41 N-helix coiled coil. Release of the drug(s) can occur immediately, gradually or at a specified time, as described above. As a result, they make contact with and bind HIV and reduce or prevent viral entry into cells.

Fusion proteins of the present invention comprise a soluble, trimeric form or version of a coiled-coil as defined in the claims, and a sufficient portion of the C-terminal end of the N peptide of HIV gp41 to bind to the C-peptide region. In one embodiment, the portion of the C-terminal end of the N-peptide comprises sufficient amino acid residues to bind to the C-peptide region and include the HIV coiled-coil cavity or hydrophobic pocket (the pocket- comprising residues of the N-peptide). The N-peptide of HIV gp41 can be that of HIV-1, HIV-2, another HIV strain or a strain from another species (e,g., simian immunodeficiency virus (SIV), feline immunodeficiency virus or Visna virus). For example, HIV-2 sequence LLRLTVWGTKNLQARVT (SEQ ID NO: 26), SIV sequence LLRLTVWGTKNLQTRVT (SEQ IID NO: 27) or a sequence comprising invariant residues in HIV-1, HIV-2 and SIV (represented LL XLTVWGXK-' UQXR-XX (SEQ ID NO: 28), wherein amino acid residues L, T, V, W, G, K, Q, and R are the single letter code used for amino acid residues and X can be any amino acid residue). Also the subject of this invention is a soluble trimeric model of the HIV gp41 hydrophobic pocket, which can be a D-peptide or an L-peptide and comprises a soluble trimeric coiled coil and a sufficient portion of the N peptide region of HIV gp41 to comprise the amino acid residues which form the pocket of the N-helix coiled-coil region of HIV gp41. The D- or L-peptide can comprise, as the soluble, trimeric coiled coil, the coiled coil of GCN4-pI_{Q}I; GCN4-pII; Moloney Murine Leukemia Virus or the ABC heterotrimer. The component which is a sufficient portion of the N peptide of HIV gp41 to comprise the amino acid residues of the pocket can comprise, for example: LLQLTVWGIKQLQARIL of HIV-1 (SEQ IID NO: 18); LLRLTVWGTKNLQARVT of HIV-2 (SEQ ED NO: 26); LRLTVWGTKNTLQTRVT of SIV (SEQ IID NO: 27) or the invariant residues of these, which are: LLXLTVWGXKXLQXRXX (SEQ ID NO: 29).

The N-helix coiled-coil of HIV gp41 can include all, part or none of the N-helix cavity. That is, a fusion protein of the present invention can comprise a trimeric form of the coiled-coil region of GCN4-pI_{Q}I and a portion of the N-peptide of HIV-1 gp41, wherein the portion of the N-peptide of gp41 comprises part, or all, or none of the N-helix cavity of HIV-I gp41.

The same strategy described herein to solve this problem for the gp41 hydrophobic pocket can be applied towards the development of soluble, trimenic models of the gp41 N-helix coiled- coil region, in general. Such trimeric models (including, for example, peptides that do not contain the pocket residues of gp41) can be used as inhibitors.

Changes can be made in the amino acid composition of the fusion protein component which is the portion from the HIV gp41 N36 peptide, to produce variants. There is no limit to the number or types of amino acid residue changes possible, provided that the trimeric state of the coiled-coil and the structure of the surface of the fusion protein corresponding to the N-peptide coiled coil of HIV gp41 are maintained. The fusion protein component which is the portion of the HIV gp4I - peptide can include all, part, or none of the N-helix cavity. For example, other parts of N51, N36, DP-107, or other regions of the HIV gp41 N-helix region can be fused to the coiled-coil region to generate trimeric (not aggregated) helical coiled-coil fusion proteins and used in the method. There is no limit to the number or types of fusion proteins that can be designed and generated, provided that the trimeric state of the coiled-coil and the structure of the surface of the fusion protein corresponding to the N-peptide coiled coil of HIV gp41 are maintained. Such fusion proteins can be designed and generated using methods known to those of skill in the art, such as evaluating heptad-repeat positions or superhelix parameters of coiled coils.

The trimer of helical hairpins (TOH) is a common feature of many viral membrane fusion proteins (Singh, M. et al. J. Mol. Biol.290,1031-1041(1999)). It has been observed in crystal structures of influenza, Ebola SV5 (simian parainfluenza virus 5), and RSV (human respiratory syncitial virus). In addition, many other members of the retrovirus, paramyxovirus, and filovirus families are predicted to contain this motif. A similar structure has been observed in the associated vertebrate vesicle fusion proteins and may be found in sperm-egg, fertilization proteins. The basic strategy described herein can be applied to any of these systems in order to inhibit fusion.

The present invention is illustrated by the following examples, which are not intended to be limiting in any way.

### Example 1 Assessment of inhibition of infectivity

### Materials and Methods

Peptide Synthesis and Purification. All peptides were chemically synthesized on a PE Biosystems 431A peptide synthesizer upgraded with feedback monitoring. The standard Fmoc/HBTU chemistry (Fields et al., 1991) was modified with DMSO/NMP resin swelling and acetic anhydride capping after every couple. The peptides were cleaved from the PE Biosystems Pal resin with Reagent K. Each peptide has an acetylated N terminus and a C-terminal amide.

The sequence of IZN17, is Ac-IKKEIEAIKKEQEAIKKKIE AIEKLLOLTVWGIKQLQARIL-NH₂ (SEQ ID NO: 32). Additional peptides that are being studied for their inhibitory activity are: IZN23 (Ac-IKKEIEAIKKEQEAIKKKIEAIEKEIEAQQHLLQLTVWGIKQLQARIL-NH₂) (SEQ ID NO: 33), IZN36 (Ac-IKKEIEAIKKEQEAIKKKIEAIEKEISGIVQQQNNLLRAIEAQQHLLQLTVWGIK QLQARIL-NH₂) (SEQ ID NO: 34) and IZN26: (Ac-YGGIKKEIEAIKKEQEAIKKKIEAIEKEIVQARQLLSGIVQQQNNLLRAIEAQQ H-NH₂ (SEQ ID NO: 14).

The coiled coil was based on a design described by Tanaka et al., but has significant alterations in the e and g positions and an I to Q substitution at an a position.

Following cleavage from the resin, each peptide was desalted over a Sephadex G-25 column (Pharmacia) and lyophilized. It was then resuspended in 5% acetic acid and purified over a Vydac C18 preparative column on a reverse phase high-performance liquid chromatography apparatus (Waters, Inc.). The peptide was eluted from the column with a water-acetonitrile gradient in the presence of 0.1 % trifluoroacetic acid and then lyophilized. The molecular weights of each peptide were validated using MALDI-TOF mass spectrometry (PerSeptive Biosystems).

Circular Dichroism. All CD measurements were performed on an Aviv 62 DS cirucular dichroism spectrometer. Standard scans were performed on 10 µM solutions of peptide in PBS (50 mM sodium phosphate, 150 mM sodium chloride [pH 7.4]) from 200 to 260 nm in a 1 cm pathlength cuvette with a 5 second averaging time. The mean residue ellipticity (θ) was calculated by dividing the raw signal by peptide concentration (M), pathlength (mm) and number of amino acids. Percent helicity was calculated according to Chen et al. (Biochemistry, 13, 1974, p3350). Thermal denaturation scans of 10 µM peptide solutions in PBS were recorded at 222 nm. The peptide was heated at two degree intervals starting at 4°C, with an equilibration time of 1.5 minutes and an averaging time of 60 seconds.

Sedimentation Equilibrium. All measurements were recorded on a Beckman XL-A analytical ultracentrifuge equipped with an An-60 Ti rotor. Lyophilized peptide was resuspended in water, and the peptide concentration was determined (Edelhoch, 1967). The solution was diluted to 100-200 µM and then dialized overnight against PBS. Following dialysis, the concentration was redetermined and the appropriate dilutions were made, using the dialysis buffer. The samples were centrifuged at speeds ranging from 19,000 to 25,000 RPM.

HIV Infectivity Assay. Inhibitory activity was determined in an HIV luciferase assay (Chen et al., 1994). Specifically, virus was made by cotransfecting 293T cells with an HIV-1 genome containing a frame-shift mutation in env and luciferase replacing the nef gene (NL43LucR-E-) along with pCMVHXB2, an expression vector with the HXB2 gp160 gene. The resultant virus is only viable for one round of infection since its genome lacks the envelope gene. The cellular debris was removed by low-speed centrifugation. The remaining viral supernatant was used to infect HOS-CD4/Fusion cells (N. Landau, National Institutes of Health AIDS Reagent Program) in the presence of the potentially inhibitory peptides. Two days post-infection, the cells were lysed and luciferase activity was monitored on a Wallac AutoLumat LB953 luminometer (Gaithersburg, MD). IC₅₀s (the peptide concentration at which half of the viral infection is inhibited) were calculated by fitting the data to a Langmuir equation [y = k/(1+[peptide]/IC₅₀)], where y = luciferase activity and k is a scaling constant.

### RESULTS

The pocket-forming region of the N peptide inhibit as a coiled-coil trimer. The x-ray crystal structure of fusogenic gp41 shows a coiled-coil trimer of N peptides surrounded by three helical C peptides. A hydrophobic pocket at the base of the N peptides, into which three hydrophobic side chains from the each of the C peptides pack, has been shown to be an important target for anti-HIV-1 compounds. Coiled coils are composed of a characteristic repeat of seven residues (designated a through g), with the first (a) and fourth (d) positions typically occupied by hydrophobic side chains. Careful attention was taken to fuse in proper coiled coil register. Here, we assayed the inhibitory activity of this chimeric molecule, and determined that this activity is reliant on the coiled coil conformation.

The pocket region of the N peptide is not required for inhibitory activity. To determine if the pocket region was required for the inhibitory potency of the chimeric N peptide molecules, an additional peptide was made, IQN26. This peptide contains 26 residues of the N peptide region N-terminal to the hydrophobic pocket. Circular dichroism studies show it is helical, and sedimentation equilibrium studies show it is slightly aggregated. It does have potent inhibitory activity. Therefore, the N peptide region, when constrained in a coiled coil formation, has inhibitory activity even in the absence of the hydrophobic pocket region.

An alternate, more stable peptide, IZN17, is a more potent inhibitor. We studied an additional IQN17 derivative, in which the IQ portion of the molecule was replaced with another trimeric designed coiled coil. This coiled coil, called 'IZ' for isoleucine zipper, is based on a design described by Tanaka, et al., but has several changes in the e and g positions and an isoleucine to glutamine substitutation at an a position. The resulting peptide is termed IZN17. IZN17 is helical and discretely trimeric at 20 µM as determined by circular dichroism and sedimentation equilibrium, respectively. Interestingly, IZN17 has an IC₅₀ of approximately 5.6 nM in the viral infectivity assay, and is therefore a much better inhibitor than IQN17. There are two potential reasons for this increase in potency. First, IZN17 is likely more stable than IQN17, and therefore stays folded at lower concentration. Both peptides melt above 100 °C, although thermal unfolding transitions can be seen in the presence of denaturant. In 2 M GuHCl, the thermal denaturation temperature of IZN17 is ten degrees higher than that of IQN17. Second, IZN17 contains two additional residues from the gp41 N peptide region, due to a coincidence in sequence between IZ and gp41. This could provide an increase in binding energy to the C peptide region of gp41.

The inhibitory activity of IZN23, IZN36 and IZN26 can be tested using known methods, such as those described herein. These peptides are likely to be potent inhibitors of HIV-1 infection.

The following Table summarizes the biophysical data and inhibitory activity for the chimeric coiled coil N peptides. The first column (θ₂₂₂ₙₘ) is circular dichroism data, representing the helicity of each peptide. The lower the number, the more helical. The second column is the melting temperature and signifies the temperature at which half of the peptide is unfolded. The next column (M_{obs}/M_{calc}) signifies the oligomeric state of the peptide, with 3.0 being a discrete trimer. The final column is the concentration at which each peptide is at half maximal inhibitory potency for viral infection.

**Table 1. Biophysical data and HIV-1 inhibitory activity**

| **PEPTIDE** | **θ₂₂₂ₙₘ** **(deg cm² dmol⁻¹)** | **Melting** **Temperature** **(°C)** | **M_{obs}/M_{calc}** | **IC₅₀ (nm)** |
|---|---|---|---|---|
| **IZN17** | ∼31,000 | >100 | 3.05 | 5.6 |
| **IZN23** | n.d. | n.d. | n.d. | n.d. |
| **IZN36** | n.d. | n.d. | n.d. | n.d. |
| **IZN26** | ∼33,000 | -82 | ∼2.87 | n.d. |

| | | | | |
|---|---|---|---|---|
| * this was determined at 50 µM Many of these values are the results of one experiment, and are therefore tentative. Results will need to be confirmed by repeating experiments. n.d. means not determined | | | | |

### Legend:

Table 1 lists all of the peptides studied. The first column gives the name of the peptide. The second column is the ellipticity at 222 nm of a 20 µM solution of peptide in PBS. The third column lists the midpoint of thermal denaturation of 10 µM solutions of peptide in PBS. The fourth column gives the ratio of the observed molecular weight of 20 µM peptide solutions in PBS by analytical ultracentrifugation to the calculated molecular weight for a monomeric peptide. The final column give the concentration of peptide at which half of viral infectivity is inhibited (IC₅₀).

## Claims

1. A soluble trimeric coiled-coil peptide comprising the following components in the following order: (a) N-terminus; (b) an isoleucine zipper (IZ) trimeric coiled-coil peptide; (c) a portion of the C-terminal end of the N-helix coiled-coil of HIV gp41 sufficient to bind to the C-peptide region; and (d) C-terminus; wherein the isoleucine zipper (IZ) trimeric coiled-coil peptide is from the coiled-coil of SEQ ID NO: 11.

2. The soluble trimeric coiled-coil peptide of daim 1 consisting of a peptide selected from: (a) IZN17 (SEQ ID NO: 32); (b) IZN23 (SEQ ID NO: 33); and (c) IZN36 (SEQ ID NO: 34).

3. A pharmaceutical composition comprising the soluble trimeric coiled-coil peptide of claim 1 or claim 2.

4. The soluble trimeric coiled-coil peptide of daim 1 or daim 2 for use in therapy or prophylaxis.

5. The soluble trimeric coiled-coil peptide of daim 1 or daim 2 for use in the treatment or prophylaxis of HIV infection.

6. The soluble trimeric coiled-coil peptide of daim 1 or claim 2 for contacting and binding HIV *in vivo* so reducing or preventing HIV viral entry into cells.

7. The soluble trimeric coiled-coil peptide of daim 1 or daim 2 for inhibiting HIV-1 infection.

## Patentansprüche

1. Ein lösliches, trimeres doppelwendelartiges (coiled-coil) Peptid, aufweisend die folgenden Komponenten in folgender Reihenfolge: (a) N-Terminus; (b) ein Isoleucin-Zipper (IZ) trimeres doppelwendelartiges Peptid; (c) eine Menge des C-terminalen Endes der N-Helix Doppelwendel des HIV gp41, ausreichend, um an die C-Peptidregion zu binden; und (d) C-Terminus; wobei das Isoleucin-Zipper (IZ) trimere doppelwendelartige Peptid von der Doppelwendel von SEQ ID NO: 11 ist.

2. Das lösliche trimere doppelwendelartige Peptid aus Anspruch 1 bestehend aus einem Peptid, ausgewählt aus: (a) IZN17 (SEQ ID NO: 32); (b) IZN23 (SEQ ID NO: 33); und (c) IZN36 (SEQ ID NO: 34).

3. Eine pharmazeutische Zusammensetzung das lösliche trimere doppelwendelartige Peptid nach Anspruch 1 oder 2 umfassend.

4. Das lösliche trimere doppelwendelartige Peptid nach Anspruch 1 oder Anspruch 2 für den Einsatz in Therapie oder Prophylaxe.

5. Das lösliche trimere doppelwendelartige Peptid nach Anspruch 1 oder Anspruch 2 für den Einsatz in der Behandlung oder Prophylaxe der HIV-Infektion.

6. Das lösliche trimere doppelwendelartige Peptid nach Anspruch 1 oder Anspruch 2 für die Kontaktaufnahme und Bindung des HIV *in vivo,* um so den HIV-viralen Eintritt in Zellen zu reduzieren oder zu verhindern.

7. Das lösliche trimere doppelwendelartige Peptid nach Anspruch 1 oder Anspruch 2 zur Hemmung einer HIV-1-Infektion.

## Revendications

1. Peptide trimère soluble en superhélice comprenant les composants suivants dans l'ordre suivant : (a) une extrémité N-terminale ; (b) un peptide trimère en superhélice à tirette à isoleucine (IZ) ; (c) une partie de l'extrémité C-terminale de l'hélice N en superhélice de gp41 du VIH suffisante pour permettre une liaison à la région du peptide C ; et (d) une extrémité C-terminale ; dans lequel le peptide trimère en superhélice à tirette à isoleucine (IZ) provient de la superhélice de SEQ ID NO : 11.

2. Peptide trimère soluble en superhélice selon la revendication 1, consistant en un peptide choisi parmi : (a) IZN17 (SEQ ID NO : 32) ; (b) IZN23 (SEQ ID NO : 33) ; et IZN36 (SEQ ID NO : 34).

3. Composition pharmaceutique comprenant le peptide trimère soluble en superhélice selon la revendication 1 ou la revendication 2.

4. Peptide trimère soluble en superhélice selon la revendication 1 ou la revendication 2, utilisable en thérapie ou en prophylaxie.

5. Peptide trimère soluble en superhélice selon la revendication 1 ou la revendication 2, utilisable dans le traitement ou la prophylaxie d'une infection par le VIH.

6. Peptide trimère soluble en superhélice selon la revendication 1 ou la revendication 2, pour entrer en contact et se lier au VIH *in vivo* pour ainsi réduire ou empêcher l'entrée du virus du VIH dans les cellules.

7. Peptide trimère soluble en superhélice selon la revendication 1 ou la revendication 2, pour inhiber une infection au VIH-1.
